# EUROPEAN PATENT SPECIFICATION

(11) **EP 0 309 456 B1**
(45) Date of publication and mention of the grant of the patent: **13.09.1995**
(21) Application number: 87903124.3
(22) Date of filing: 15.04.1987
(51) Int. Cl.: A61K 35/28, C12N 5/08

(54) **Stromal tissue**
Stromales Gewebe
Tissu stromal

(30) Priority: 18.04.1986 US 853569; 03.04.1987 US 36154; 14.04.1987 US 38110
(43) Date of publication of application: 05.04.1989
(73) Proprietor: ADVANCED TISSUE SCIENCES, INC. (a Delaware Corporation), Elmsford New York 10523 (US)
(72) Inventor: NAUGHTON, Brian, A. R.D. 2 Box B339, Yorktown Heights, NY 10598 (US); NAUGHTON, Gail, K. R.D. 2 Box B339, Yorktown Heights, NY 10598 (US)
(74) Representative: Jones, Andrée Zena
(86) International application number: US8700869
(87) International publication number: WO8706120

(56) References cited:
- US-A- 3 997 396
- US-A- 4 024 020
- US-A- 4 144 126
- US-A- 4 154 652
- US-A- 4 184 922
- US-A- 4 228 243
- BIOLOGICAL ABSTRACT, vol. 62, October 15, 1976, pages 4275, abstract 43283, D. REDELMAN et al.
- EXPERIMENTAL HEMATOLOGY, vol. 12, no. 6, 1984, page 403, abstract 186, S. BOSWELL et al.

## Description

The present invention relates to the production of a three-dimensional living stromal tissue in vitro and its use for culturing bone marrow, skin, liver and many other cell types and tissues. The cultured cells may be used for transplantation, cytotoxicity testing and in vitro testing of particular disease states.

Processes for transplanting bone marrow are known. Generally, bone marrow transplants are performed on patients who suffer from a disease, such as cancer, which destroys healthy bone marrow cells or depresses their functional ability. In addition, treatments such as chemotherapy or radiation therapy adversely affect the bone marrow even in cases where the bone marrow has not been directly affected by the disease being treated. Known methods of bone marrow transplantation suffer from a number of disadvantages. One major cause of bone marrow transplant rejection is the graft versus host reaction which occurs when the bone marrow removed from one person is transplanted into another person. Another major cause of bone marrow transplant failure is vasoocclusive disease resulting from the formation of marrow emboli. procedures for the removal and storage of a persons' marrow prior to combined chemotherapy and radiation and reinfusion of that marrow currently exist (i.e. autologous transplant). However, the patient often suffers from the recurrence of the disease even if engraftment occurs since the marrow was already diseased when first removed.

One aspect of the invention provides a three-dimensional living stromal tissue prepared in vitro, comprising stromal cells and connective tissue proteins secreted by the stromal cells, grown on a biocompatible non-living three-dimensional support, wherein the stromal cells are attached to and encircle the support to form a three-dimensional structure.

Further aspects provide a method of cell culture comprising bone marrow, skin or liver cells cultured on the living stromal tissue, and corresponding uses of said cultured cells.

The invention describes a three-dimensional living stromal tissue for growing cells in culture. The growth of cells in the presence of this living stromal tissue may be further enhanced by adding proteins, glycoproteins, glycosaminoglycans, a cellular matrix, and other materials to the stromal tissue itself or by coating a non-living three-dimensional support upon which the stromal tissue grows, with these materials. The use of a three-dimensional living stromal tissue allows the cells to grow in multiple layers, thus creating the three-dimensional, cell culture system of the present invention. The three-dimensional living stromal tissue can be used to create three-dimensional, cell cultures systems for bone marrow, skin, liver, and many other cell types and tissues. In one embodiment of this invention, the three-dimensional cell culture system may be transferred onto or into a living organism.

The three-dimensional support is preferably in the form of a mesh. Prepared supports may be preserved for later use in a three-dimensional, physiologic, cell culture system. In an embodiment for growing hematopoietic bone marrow cells, the mesh is prepared by growing a layer of stromal marrow cells on the mesh until the stromal marrow cells reach subconfluency.

The invention will now be described by way of example only with reference to its applications to bone marrow, it being understood however, that the invention is applicable to many other cell types and tissues.

A method of culturing bone marrow for use in treating a person whose bone marrow has been destroyed or lost its functional ability comprises the steps of:
I. obtaining a bone marrow sample from a donor; and then
II. culturing the bone marrow sample in vitro to produce a cultured bone marrow containing hematopoietic stem cells having marrow repopulating activity, a portion of which may be cryopreserved for later use; and then
III. transplanting the cultured bone marrow containing hematopoietic stem cells into the person to restore hematopoiesis in the person.

Also, there is provided a use for treating diseases or conditions which have destroyed healthy bone marrow cells or have depressed their functional ability. This is especially effective in the treatment of hematological malignancies and other neoplasias which metastasize to the bone marrow.

One embodiment of the present invention comprises the steps of: aspirating a small amount of bone marrow from a healthy patient; cryopreserving said cells; culturing the bone marrow cells in vitro to increase the number of bone marrow cells to a number sufficient for autologous transplantation, for instance reinfusion, which is greater than the number originally removed from the patient; and then reinfusing the bone marrow cells into the patient. According to another embodiment of the present invention, the bone marrow cells are cryopreserved or frozen immediately after aspiration from the patient and are subsequently thawed and replicated. According to another embodiment of the present invention, an allogenic bone marrow transplant is performed by aspirating bone marrow from one person, replicating the bone marrow cells in vitro and then reinfusing the replicated bone marrow cells into another person.

The in vitro bone marrow replication system also provides a means to monitor a patient in regard to those conditions that affect the bone marrow. For example, a small sample of bone marrow is aspirated from a treated cancer patient and replicated in the in vitro system of the present invention, in order to check for a recurrence or metastasis of the original malignancy.

The in vitro bone marrow replication system is also adaptable for cytotoxicity testing of pharmaceuticals, food additives, health products, anti-neoplastic agents, and carcinogens. A proportional, scaled-down dose of the agent to be tested is added to the in vitro bone marrow system of the invention and its effect on various cell types noted. Cell types other than bone marrow cells are substituted to test a particular agent, as required.

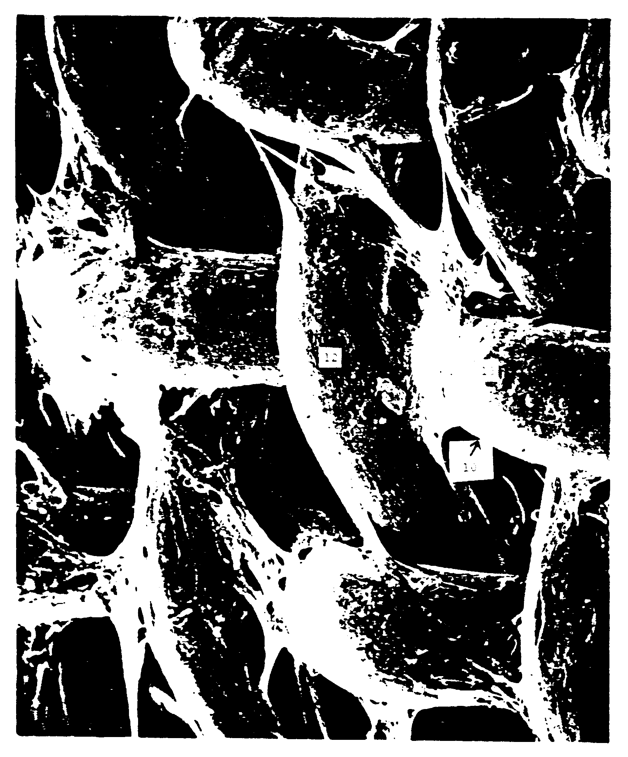

The invention may be better understood by reference to the single figure of the following drawings showing a scanning electron photomicrograph at a magnification of 1.65 thousand times showing a meshwork with bone marrow stromal cells growing thereon.

Specific embodiments of the present invention will now be described by way of example only.

Referring now to the single figure of the drawings, there is shown a meshwork 10 useful in the present invention. The meshwork 10 contains warp threads 12 and woof threads 13. As shown in the figure, stromal cells 14 can be seen growing on the threads 12, 13.

### Replication and Cyropreservation of Bone Marrow for Use in Bone Marrow Transplantations

In one embodiment the present invention is directed to the culture of bone marrow cells for the manufacture of a transplant for treating diseases or conditions which destroy healthy bone marrow cells or depress their functional ability. This is effective especially in the treatment of hematological malignancies and other neoplasias which metastasize to the bone marrow. This invention is also effective in treating patients whose bone marrow has been adversely affected by chemotherapy and/or radiation therapy necessitated by a disease which does not directly affect the bone marrow. The present invention further provides a means for removing and preserving bone marrow cells from a healthy patient and then replicating and reinfusing the bone marrow cells should the patient develop an illness which either destroys the bone marrow directly or whose treatment adversely affects the marrow.

In accordance with the present invention, a small amount (10-15 cc bone marrow/peripheral blood suspension) is aspirated from the iliac crest of a donor. The results of the process are optimal if: 1. The individual is under 40 years of age at the time his/her marrow is cryopreserved, 2. the patient is disease-free. This procedure may prove beneficial to certain patients with metastatic disease or hematological malignancies if "purging" of malignant cells by physical or chemotherapeutic means can be performed prior to culturing. At present, these methods are most efficient when small numbers of cells are used, a fact which might lend itself well to the following procedures. Methods of aspirating bone marrow from a donor are well known in the art. Examples of apparatus and processes for aspirating bone marrow from a donor can be found in U.S. Patents 4,481,946 and 4,486,188.

The bone marrow removed from the donor is then cultured, i.e. replicated, or preserved for replication at a later date. If the bone marrow is to be preserved, the bone marrow can be incrementally frozen using computerized cryotechnological equipment. Fresh bone marrow/blood suspension is aliquoted in equal volumes into sterile Nunc tubes and placed in a beaker of crushed ice until the cryopreservation chamber is brought to a similar temperature (4°C). Immediately prior to specimen insertion into the chamber, a solution is added to each Nunc tube using sterile technique, so that the cryoprotectants, dimethylsulfoxide and glycerol, will be final concentrations of 7% and 5% respectively. The freezing program is initiated immediately after introduction of the specimen. Freezing program number 1 on the CryoMed Model Number 1010 controller is used.

Using this technique, the cellular viability after freezing and rapid thawing in an 80°C water bath exceeds 90% via the trypan blue exclusion method. In addition greater than 80% of the original colony forming unit culture (CFU-C) may be recovered after freezing. Examples of systems for freezing bone marrow and biological substances in accordance with a precalculated temperature and time curve are disclosed in U.S. patents 4,107,937 and 4,117,881. Preferably, the bone marrow cells are stored in the liquid phase of liquid nitrogen at a temperature of -196°C at which temperature all cellular metabolic activity has ceased.

After thawing, the bone marrow cells are reinfused into an individual or are replicated, and then reinfused.

The present invention has several advantages to a patient in need of a bone marrow transplant. If the patient is receiving his or her own cells, this is called an autologous transplant. Such a transplant has little likelihood of rejection. Autologous transplants eliminate a major cause of bone marrow transplant rejection, that is, the graft vs. host reaction. Further, when grown in culture, hematopoietic cells may be easily separated by physical manipulation and/or enzyme treatment. This diminishes the risk of vasoocclusive disease resulting from marrow emboli. In addition, the present invention allows more aggressive treatment of neoplastic disorders with chemotherapeutic agents and radiation. Presently, the extent of these treatments is often limited by bone marrow toxicity.

### In Vitro Bone Marrow Replication System

The method of the present invention comprises the step of replicating the bone marrow cells in vitro, in a system comparable to physiologic conditions. Moreover, the bone marrow cells replicated in this system include all of the cells present in normal bone marrow, assuming all cell types were present in the original bone marrow inoculum.

The bone marrow cells, either obtained directly from the donor or retrieved from cryopreservative storage, are first separated from their reticulum by physical means. The bone marrow cells are then grown in co-cultures with stromal components of normal marrow, which includes fibroblasts, macrophages, reticular cells, and adipocytes, on a three-dimensional support. Factors derived from media of splenic and/or hepatic (liver) macrophage cultures or from subsets of stromal cells may also be added to the culture.

Although marrow cells are capable of limited growth when cultured alone, long term growth of these cultures is possible only if stromal cells or their secretory products are present. See Long-Term Bone Marrow Culture, D.G. Wright & J.S. Greenberger, eds., A.R. Liss, New York, (1984).

The present invention seeks to maximize the proliferation of multipotential hematopoietic stem cells which have the capability of repopulating bone marrow when the bone marrow has been destroyed by intrinsically or environmentally-mediated disease or by the treatment of such disease with chemotherapy and/or radiation. Stem cells which have marrow repopulating activity (MRA) have been shown to persist and replicate in the long term bone marrow cultures. However, stem cells, hemopoietic progenitor cells, hemopoietic precusor cells all replicate and proliferate in the system of the present invention. Furthermore, differentiation can proceed in this system in a physiologic manner. For example, erythroid, myeloid, lymphoid, macrophagic, and megakaryocytic colonies, can continuously arise in the same culture system using the systems as taught by the present invention.

According to a preferred embodiment of the present invention, hematopoietic stem cell growth is accomplished as follows:

### 1. establishment of the stromal support layer

Marrow suspensions are centrifuged at 3000 x g for 20 minutes and the white base of cells containing macrophages, fibroblasts, adipocytes, mononuclear blood cells, reticular cells, endothelial cells and other resident cells is removed. The cells are suspended in a medium called Roswell Park Memorial Institute medium number 1640 or simply "RPMI 1640". The RPMI 1640 is purchased from GIBCO Incorporated of Grand Island, New York, USA. The RPMI 1640 supplemented with 10% fetal bovine serum, 10% horse serum, hydrocortisone hemisuccinate, and appropriate antibiotics.

10⁶ of these cells are plated onto sterile nylon mesh from the Tetko Corp. of New York, New York, USA in a petri dish. This mesh has been pre-cut to conform to the inside dimensions of a 25mm² plastic culture flasks. The mesh has a sieve area of 400µm² and a fiber diameter of 100µm.

The inoculated mesh is then wound and placed into the culture flask containing 5 milliliters of media as previously described. The mesh unwinds inside the culture flask and completely covers the bottom of the flask. The cultures arc grown at 37°C in 5% C0₂ in ambient air at a relative humidity in excess of 90%. Stromal cells which are predominantly fibroblasts first grow along and encircle all of the nylon fibers before beginning to grow into the mesh openings. This process takes approximately 14 to 18 days. The degree of subconfluency of the stromal cells, should be consistent with that seen in the figure of the drawing, prior to the inoculation of hematopoietic cells.

Suspended stromal cells can be cryopreserved using the same technique as previously described for bone marrow cells. For cryopreservation of sub-confluent cells on the mesh, the nylon mesh must be rolled and inserted into the Nunc tube containing RPMI 1640 medium with the cryoprotectants dimethylsulfoxide and glycerol in final concentrations of 5% and 15% respectively. Freezing of the stromal cells on the mesh can be accomplished at initial cooling rates of -1°C/min from +1°C to -40°C. A cooling rate of -2 to -3°C/min is optimum is utilized until the end stage temperature of -84°C is achieved. Approximately 20-25% of the stromal cells will detach from the nylon mesh.

### 2. inoculation with hematopoietic cells

Bone marrow cells are suspended in a modified Fischer'S or McCoy's 5A medium supplemented with 5-10% fetal bovine serum and 5-10% horse serum, vitamins, hydrocortisone, glutamine and antibiotics. These cells may either be fresh or derived from a formerly cryopreserved sample which has been rapidly thawed in an 80°C hot water bath. 2 to 5 X 10⁶ cells are inoculated onto subconfluent stromal cell meshworks in 25 mm² plastic culture flasks and grown at 33 to 34°C at 5% C0₂ in ambient air. The relative humidity of these cultures must be greater than 90%. After 3 days the culture temperature is raised to 35 to 37°C.

Hematopoietic cells grow in the natural pockets formed by the subconfluent stromal cells and the progenitor cells remain in the adherent layer of cells. The adherent layer are those cells attached directly to the mesh or those connected indirectly by attachment to cells that are themselves attached directly to the mesh. After 4 to 5 days, mature granulocytes, mononuclear cells, and erythrocytes appear in the non-adherent layer as observed by cytospin preparation. After 7 to 10 days, numerous hematopoietic colonies can be observed in the interstices of the mesh and are morphologically consistent with CFU-C, mixed colonies, and lymphoid colonies. Megakaryocytic growth is limited but may be observed in this matrix as well. An average culture will produce 450 to 950 CFU-C per week.

Cultures which consist of stromal cells and hematopoietic cells derived from the same individual (autologous) must be fed twice weekly. Cultures which consist of a patient's bone marrow which has been inoculated onto a stromal cell meshwork derived from another individual(s) (allogeneic) must be fed three times per week to insure adequate depopulation of mature immunocompetent cells from the non-adherent layer.

### 3. variations in the in vitro bone marrow replication system

### Enhancing the Growth of Marrow Stromal Cells

The primary rate limiting factor in the growth of marrow stromal cells is the relatively low mitotic index of the fibroblasts, included among the marrow stromal cells. The growth of these cells and their deposition of extracellular matrix components may be enhanced by adding: 1. hydrocortisone hemisuccinate and/or 2. self-regulating growth factors derived from the medium of cultured human fetal fibroblasts which have a high rate of cell division.

Attachment and growth of fibroblasts on the mesh can also be enhanced by: 1. pre-coating the mesh with solubilized type I-IV collagen, or 2. using a mesh which is coated or embedded with collagen secreted by fetal human fibroblasts or by adult fibroblasts (hereinafter referred to as "growth enhancing fibroblasts") which have been subsetted by their ability to synthesize certain collagen types. In this regard, the growth enhancing fibroblasts are lifted by mild trypsinization from the mesh upon reaching confluency (5 to 7 days for fetal human fibroblasts and 14 to 18 days for adult fibroblasts respectively) and may either be 1. inoculated with stromal marrow cells as previously described or 2. cryopreserved for future use.

In one embodiment of the invention, growth enhancing fibroblasts that are synthesizing collagen and other extracellular matrix components are grown on the mesh until they reach subconfluency. A mixture of both hematopoietic and stromal bone marrow cells are then inoculated onto the subconfluent growth enhancing fibroblast meshwork.

The methods for growing, subsetting, and cryopreserving growth enhancing fibroblasts are as follows:

### a. Culture of Growth Enhancing Fibroblasts

Fibroblasts are grown in RPMI 1640 supplemented with 2-10% fetal bovine serum or 2-10% horse serum to which 1µg/mL hydrocortisone hemisuccinate and 2µg/mL gentamycin, penicillin, streptomycin and fungizone have been added. Cultures are grown at 5% C0₂ in ambient air at 37°C with a relative humidity greater than 90%.

### b. Subsetting Growth Enhancing Fibroblasts

5.0 X 10⁶ fibroblasts derived from the buffy coat of a bone marrow suspension, dermal fibroblasts, or fibroblasts derived from cadaver livers are plated onto microtiter wells (1mm²) and grown to confluency. These cells are lifted from the culture wells by repeated washings usually four to five times with Hank's balanced salt solution without Ca⁺⁺ or Mg⁺⁺. The matrix remaining on the microtiter plates is examined by indirect immunofluorescence utilizing monoclonal antibodies to various matrix components and fluorescein isothiocyanate-labelled, rabbit anti-mouse immunoglobulin G to ascertain the collagen types present. The suspended cells are treated with monoclonal antibodies directed against collagen types I-IV, elastin, tropoelastin, and fibronectin to isolate sub-populations of cells capable of synthesizing each product. The cells are treated with guinea pig complement which will damage or destroy those cells to which monoclonal antibody is attached. The viable cells are re-plated onto microtiter wells as previously described, are grown to confluency, and lifted. The efficiency of the isolation technique is then verified by examining the matrix secreted by those cells with monoclonal antibodies and indirect immunofluorescence.

For optimal growth of hematopoietic cells the matrix should contain collagen types III, IV and I in an approximate ratio of 6:3:1.

### c. Cryopreservation of Growth Enhancing Fibroblasts

Growth enhancing fibroblasts can be cryopreserved using the same techniques as previously described for stromal cells. Like the stromal cells, some of the growth enhancing fibroblasts will also detach from the mesh during freezing. This matrix, however, still contributes to the attachment of marrow stromal cells and therefore diminishes the time required for the establishment of a matrix conductive to hematopoietic cell growth.

### Inoculation With Mononuclear Cells

To enhance the long-term growth of bone marrow cultures, peripheral blood mononuclear cells are prepared from a heparinized suspension using Ficoll-hypague or Percoll. Peripheral blood cells and bone marrow hematopoietic cells are derived from the same individual (autologous). These should be removed via venipuncture and cryopreserved at the time the bone marrow specimen is taken. Additional peripheral blood cells could be procured from the diseased patient if needed during the culturing procedure. However, if metastatic disease is suspected, the sample must first be subjected to purging, as mentioned previously. 5 x 10⁵ to 10⁶ mononuclear cells (the monocyte subpopulation is the preferred cell type within the mononuclear cell layer for this step) are inoculated onto meshworks 4 to 5 days after the initial inoculation with bone marrow hematopoietic cells and every third week thereafter. This procedure enhances hematopoiesis by 10 to 13% as observed on a weekly basis.

### Perpetuation Of The Culture And Banking Of Progenitor Cells

In our experience, confluent stromal cell cultures will not or at best poorly support hematopoiesis. indefinite growth of human hematopoietic progenitors is possible if they are provided with the necessary stromal-derived growth/regulatory factors.

For example, the initial marrow sample is divided into a number of aliquots containing approximately 10⁶ hematopoietic cells. Each of these is inoculated onto a sub-confluent stromal cell meshwork. The cultures are monitored by direct observation with an inverted phase microscope and differential counts of the non-adherent cells as seen on the cytospin preparation after each feeding. Prior to reaching confluency, the cultures are treated with collagenase and placed under mild ultrasonication for approximately 6-10 minutes. Hematopoietic cells and stromal cells are separated by density gradient methods. The hematopoietic cells are counted using a hemacytometer and approximately 50% are cryopreserved using methods described previously. The remaining 50% of the hematopoietic cells are divided into aliquots consisting of approximately 10⁶ cells and are inoculated onto sub-confluent stromal cell cultures which have been staggered and grown in parallel. When these begin to reach confluency, the same procedure is performed.

This technique: 1. perpetuates the growth of hematopoietic cells by providing a microenvironment which produces the required growth factors and, 2. forms a continuous bank where hematopoietic progenitors may be deposited until the numbers suitable for engraftment are achieved.

### Modulation Of Hematopoietic Cell Growth With Cell Products

The technology presently exists to sub-culture the various cellular components of human marrow as separate cultures. Macrophages, reticular cells, adipocytes, and fibroblasts may be grown separately and their secretory activity modified by treatment with various agents. Modulation of fibroblasts activity has been described previously.

Hematopoiesis in long-term human marrow cultures on the three dimensional meshwork may also be modulated by secretions of extramedullary macrophages (Kupffer cells) when grown in culture in the following manner. Kupffer cells are separated from their organ stroma after pronase digestion. Briefly, tissue specimens will be incubated for 1 hour in pronase solution [0.2% pronase (Calbiochem) and Geys' Balanced Salt Solution (BSS)] while being gently agitated. The pH of the solution is maintained at 7.3 to 7.5 with 1N NaOH. Deoxyribonuclease (0.5 mg) (Calbiochem) is added at 30 minute intervals during the above procedure and the resultant cell suspension is filtered and centrifuged at 350 x G for 10 minutes. The pellet is resuspended in Geys' BSS and the littoral cells (macrophages and endothelial cells) are separated from the cellular debris and mature blood cells using a Percoll (Pharmacia) gradient. The resultant cell fraction is washed 3 x 3 minutes with a modified Dulbecco's medium enriched with 10% fetal bovine serum and plated onto plastic culture dishes at a volume containing 3 to 4 x 10⁶ cells.

After incubation for 1 day, the non-adherent cells are removed by washing with the culture medium and the adherent cell are maintained at 33°C in a gas mixture consisting of 6% C0₂ in room air at over 80% relative humidity. The growth and/or secretory activity of these cells can be stimulated by: 1. varying the C0₂ : 0₂ ratio, 2. treating the cultures with latex beads, 3. treating the cultures with silica, 4. adding prostaglandin E₂, E₁ or F_{2α} to the medium, 5. supplementing the medium with interleukin 1 or interleukin 2. Macrophage secretory products may be modulated by these procedures/agents.

The medium conditioned with the secretory products of these macrophages may be used to supplement the long-term bone marrow culture erythropoietic/granulopoietic ratio in much the same manner as occurs in vivo.

The process of the present invention has several advantages to a patient in need of a bone marrow transplant.

### Use of In Vitro Bone Morrow Replication System to Monitor a Patient's Condition

In a patient with metastatic (e.g. Cancer) or other diseases, it is often efficacious to monitor the patient's condition by aspirating a portion of the patient's bone marrow and examining the sample. In this manner, a metastasis or recurrence may be detected before it is clinically obvious. Patients with other conditions that are detectable by examining bone marrow cells may also be monitored in this way.

The long-term growth of cells in an aspirated bone marrow specimen using the bone marrow replication system of the present invention enhances the likelihood of the detection of clonal metastatic cells and hematopoietic cells with chromosomal abnormalities. These cells may escape detection in a conventional smear of freshly aspirated (uncultured) bone marrow.

### Use of In Vitro Bone Marrow Replication System in Cytotoxicity System

The cytotoxicity to bone marrow of pharmaceuticals, anti-neoplastic agents, carcinogens, food additives, and other substances to bone marrow is tested by utilizing the in vitro bone marrow replication system of the present invention.

First, stable, growing cultures of bone marrow cells (including both stromal and hematopoietic cells) are established. Then, the cultures are exposed to varying concentrations of the test agent. After incubation with the test agents, the culture are examined by phase microscopy to determine the highest tolerated dose (HTD) - the concentration of test agent at which the earliest morphological abnormalities appear. Cytotoxicity testing can be performed using a variety of supravital dyes to assess cell viability in this three-dimensional system, using techniques well-known to those skilled in the art. The HTD determination provides a concentration range for further testing.

Once a testing range is established, varying concentrations of the test agent can be examined for their effect on viability, growth, and/or morphology of the different cell types constituting the bone marrow culture by means well known to those skilled in the art.

Other three-dimensional cell culture systems as disclosed in the present invention may be adopted for use in cytotoxicity testing.

### The Establishment of a Three-Dimensional Cell Culture System

The present invention discloses a three-dimensional living stromal tissue and its use as the framework for a three-dimensional, multi-layer cell culture system. In previously known tissue culture systems, the cells were grown in a monolayer. U.S. 3,997,396 discloses a method of propagating and maintaining cells of the external surface of a hollow fibre membrane. Cells grown on a three-dimensional living stromal tissue in accordance with the present invention grow in multiple layers, forming a cellular matrix. This three-dimensional cell culture system approaches physiologic conditions found in vivo to a greater degree than previously described monolayer tissue culture systems. In one embodiment of this invention, the three-dimensional cell culture system may be transferred onto or into a living organism.

The three-dimensional living stromal may be grown on any three-dimensional biocompatible non-living support material that: 1. allows cells to attach to it (or can be modified to allow cells to attach to it) and 2. allows cells to grow in more than one layer. The three-dimensional support is a mesh in a preferred embodiment of the present invention.

It is contemplated that the three-dimensional cell culture system is applicable to bone marrow, skin, or liver, and any other cell types and tissues. For example, a three-dimension skin cell culture system is produced as follows:
1. Fibroblast are allowed to attached to a mesh and grown for 7-9 days, depositing collagen types I and III, as described previously in regard to the growth enhancing fibroblast used in the in vitro bone marrow replication systems;
2. Melanocytes are plated onto the treated mesh and are allowed to grow for 5 days;
3. Keratinocytes are inoculated onto subconfluent melanocytes.

## Claims

1. A three-dimensional living stromal tissue prepared in vitro, comprising stromal cells and connective tissue proteins secreted by the stromal cells, grown on a biocompatible non-living three-dimensional support, wherein the stromal cells are attached to and encircle the support to form a three-dimensional structure.

2. The living stromal tissue of claim 1 in which the stromal cells are fibroblasts.

3. The living stromal tissue of claim 1 in which the stromal cells are a combination of fibroblasts and endothelial cells, macrophages, reticular cells, mononuclear blood cells or adipocytes.

4. The living stromal tissue of any preceding claim in which the support is pre-coated with collagen.

5. The living stromal tissue of any preceding claim in which the support is a mesh.

6. The living stromal tissue of claim 5 wherein the mesh is formed of nylon.

7. A three-dimensional cell culture comprising cells cultured on a living stromal tissue according to any preceding claim, the cells being bone marrow, skin or liver cells.

8. A three-dimensional bone marrow culture comprising hematopoietic cells cultured on a living stromal tissue according to any of claims 1 to 6.

9. A three-dimensional skin culture comprising melanocytes and keratinocytes cultured on a living stromal tissue according to any of claims 1 to 6.

10. The three-dimensional skin culture of claim 9 in which the stromal cells are confluent.

11. A three-dimensional liver culture comprising hepatocytes cultured on a living stromal tissue according to any of claims 1 to 6.

12. A method for culturing cells in vitro comprising:
(a) inoculating bone marrow, skin or liver cells onto a living stromal tissue according to any of claims 1 to 6; and
(b) culturing the inoculated living stromal tissue so that the inoculated cells grow.

13. A method for culturing bone marrow cells in vitro comprising:
(a) inoculating hematopoietic cells onto a living stromal tissue according to any of claims 1 to 6; and
(b) culturing the inoculated living stromal tissue so that the inoculated cells grow.

14. A method for culturing skin cells in vitro, comprising:
(a) inoculating melanocytes and keratinocytes onto a living stromal tissue according to any of claims 1 to 6; and
(b) culturing the inoculated living stromal tissue so that the inoculated cells grow.

15. The method according to claim 14 in which the living stromal tissue comprises confluent stromal cells.

16. A method for culturing liver cells in vitro comprising:
(a) inoculating hepatocytes onto a living stromal tissue according to any of claims 1 to 6; and
(b) culturing the inoculated living stromal tissue so that the inoculated cells grow.

17. The use of bone marrow, skin or liver cells cultured in vitro for the manufacture of a transplant; in which the culturing comprises:
(a) inoculating bone marrow, skin or liver cells onto a living stromal tissue according to any of claims 1 to 6; and
(b) culturing the inoculated living stromal tissue so that the inoculated cells grow.

18. The use according to claim 17 wherein the cells are for transplantation of bone marrow cells prepared by culturing hematopoietic cells in vitro so that the hematopoietic cells grow.

19. The use according to claim 17 of skin cells prepared by culturing melanocytes and keratinocytes in vitro so that the melanocytes and keratinocytes grow.

20. The use according to claim 17 of liver cells prepared by culturing hepatocytes in vitro for the manufacture of a transplant.

21. The use of living stromal tissue according to any of claims 1 to 6 for the manufacture of a transplant.

22. A method for testing the cytotoxicity of a test substance comprising:
(a) exposing a three-dimensional cell culture to the test substance, in which the three-dimensional cell culture comprises bone marrow, skin or liver cells grown on a living stromal tissue according to any of claims 1 to 6; and
(b)determining the effect of the test substance by observing any changes in the cells.

23. The method for testing the cytological effect of a test substance according to claim 22 in which the cells are hematopoietic cells.

24. The method for testing the cytotoxicity of a test substance according to claim 23 in which the cells are melanocytes and keratinocytes.

25. The method for testing the cytotoxicity of a test substance according to claim 23 in which the cells are hepatocytes.

26. A method for detecting metastatic cells comprising:
(a) obtaining a sample of bone marrow, skin or liver cells;
(b) inoculating cells from the sample onto a living stromal tissue according to any of claims 1 to 6;
(c) culturing the inoculated living stromal tissue in a nutrient medium so that the inoculated cells grow; and
(d) determining the presence of metastatic cells in the cells grown in culture.

27. The method for detecting metastatic cells according to claim 27 in which the cells are hematopoietic cells.

28. The method for detecting metastatic cells according to claim 27 in which the cells are melanocytes and keratinocytes.

29. The method for diagnosing a chromosomal abnormality according to claim 27 in which the cells are hepatocytes.

30. A method of preparing in vitro a three-dimensional living stromal tissue comprising stromal cells and connective tissue proteins secreted by the stromal cells, which comprises growing the stromal tissue on a biocompatible non-living three-dimensional support, whereby the stromal cells are attached to and encircle the support to form a three-dimensional structure.

31. The method of claim 30 in which the stromal cells are fibroblasts.

32. The method of claim 30 in which the stromal cells are a combination of fibroblasts and endothelial cells, macrophages, reticular cells, mononuclear blood cells or adipocytes.

33. The method of any of claims 30 to 32 in which the support is pre-coated with collagen.

34. The method of any of claims 30 to 33 in which the support is a mesh.

35. The method of claim 34 wherein the mesh is formed of nylon.

## Patentansprüche

1. Ein in vitro hergestelltes, Stromazellen und von den Stromazellen ausgeschiedene Bindegewebsproteine umfassendes dreidimensionales lebendes Stromagewebe, gezüchtet auf einem biokompatiblen nichtlebenden dreidimensionalen Träger, dadurch gekennzeichnet, daß die Stromazellen auf dem Träger so festhaften und den Träger so umschließen, daß eine dreidimensionale Struktur entsteht.

2. Das lebende Stromagewebe gemaß Anspruch 1, dadurch gekennzeichnet, daß es sich bei den Stromazellen um Fibroblasten handelt.

3. Das lebende Stromagewebe gemäß Anspruch 1, dadurch gekennzeichnet, daß es sich bei den Stromazellen um eine Kombination aus Fibroblasten und Endothelzellen, Makrophagen, Retikulumzellen, mononukleären Blutzellen oder Fettzellen handelt.

4. Das lebende Stromagewebe gemäß einem der vorgenannten Ansprüche, dadurch gekennzeichnet, daß der Träger mit Kollagen vorbeschichtet ist.

5. Das lebende Stromagewebe gemäß einem der vorgenannten Ansprüche, dadurch gekennzeichnet, daß es sich bei dem Träger um ein Netz handelt.

6. Das lebende Stromagewebe gemaß Anspruch 5, dadurch gekennzeichnet, daß das Netz aus Nylon besteht.

7. Eine dreidimensionale Zellkultur umfassend auf einem lebenden Stromagewebe gemäß einem der vorgenannten Ansprüche kultivierte Zellen, dadurch gekennzeichnet, daß es sich bei den Zellen um Knochenmarkzellen, Hautzellen oder Leberzellen handelt.

8. Eine dreidimensionale Knochenmarkkultur umfassend auf einem lebenden Stromagewebe gemäß einem der Ansprüche 1 bis 6 kultivierte blutbildende Zellen.

9. Eine dreidimensionale Hautkultur umfassend auf einem lebenden Stromagewebe gemäß einem der Ansprüche 1 bis 6 kultivierte Melanozyten und Keratinozyten.

10. Die dreidimensionale Hautkultur gemäß Anspruch 9, dadurch gekennzeichnet, daß die Stromazellen zusammenwachsend sind.

11. Eine dreidimensionale Leberkultur umfassend auf einem lebenden Stromagewebe gemäß einem der Ansprüche 1 bis 6 kultivierte Leberzellen.

12. Ein Verfahren zur In-vitro-Kultivierung von Zellen, umfassend:
(a) Beimpfen von lebendem Stromagewebe gemäß einem der Ansprüche 1 bis 6 mit Knochenmarkzellen, Hautzellen oder Leberzellen;
und
(b) Kultivieren des beimpften lebenden Stromagewebes, so daß die beimpften Zellen wachsen.

13. Ein Verfahren zur In-vitro-Kultivierung von Knochenmarkzellen, umfassend:
(a) Beimpfen von lebendem Stromagewebe gemäß einem der Ansprüche 1 bis 6 mit blutbildenden Zellen;
und
(b) Kultivieren des beimpften lebenden Stromagewebes, so daß die beimpften Zellen wachsen.

14. Ein Verfahren zur In-vitro-Kultivierung von Hautzellen, umfassend:
(a) Beimpfen von lebendem Stromagewebe gemäß einem der Ansprüche 1 bis 6 mit Melanozyten und Keratinozyten;
und
(b) Kultivieren des beimpften lebenden Stromagewebes, so daß die beimpften Zellen wachsen.

15. Das Verfahren gemäß Anspruch 14, dadurch gekennzeichnet, daß es sich bei dem lebenden Stromagewebe um zusammenwachsende Stromazellen handelt.

16. Ein Verfahren zur In-vitro-Kultivierung von Leberzellen, umfassend:
(a) Beimpfen von lebendem Stromagewebe gemäß einem der Ansprüche 1 bis 6 mit Leberzellen;
und
(b) Kultivieren des beimpften lebenden Stromagewebes, so daß die beimpften Zellen wachsen.

17. Die Verwendung von in vitro kultivierten Knochenmarkzellen, Hautzellen oder Leberzellen zur Herstellung eines Transplantats; dadurch gekennzeichnet, daß das Kultivieren folgendes umfaßt:
(a) Beimpfen von lebendem Stromagewebe gemäß einem der Ansprüche 1 bis 6 mit Knochenmarkzellen, Hautzellen oder Leberzellen;
und
(b) Kultivieren des beimpften lebenden Stromagewebes, so daß die beimpften Zellen wachsen.

18. Die Verwendung gemäß Anspruch 17, wobei die Zellen der Transplantation von Knochenmarkzellen gewonnen durch In-vitro-Kultivierung von blutbildenden Zellen dienen, so daß die blutbildenden Zellen wachsen.

19. Die Verwendung von Hautzellen gemäß Anspruch 17, gewonnen durch In-vitro-Kultivierung von Melanozyten und Keratinozyten, so daß die Melanozyten und Keratinozyten wachsen.

20. Die Verwendung von Leberzellen gemäß Anspruch 17, gewonnen durch In-vitro-Kultivierung von Leberzellen zur Herstellung eines Transplantats.

21. Die Verwendung von lebendem Stromagewebe gemäß einem der Ansprüche 1 bis 6 zur Herstellung eines Transplantats.

22. Ein Verfahren zur Prüfung der Zytotoptät einer Testsubstanz, umfassend:
(a) Aufbringen der Testsubstanz auf eine dreidimensionale Zellkultur, dadurch gekennzeichnet, daß es sich bei der dreidimensionalen Zellkultur um Knochenmarkzellen, Hautzellen oder Leberzellen handelt, gezüchtet auf einem lebenden Stromagewebe gemäß einem der Ansprüche 1 bis 6;
und
(b) Ermitteln der Wirkung der Testsubstanz durch Beobachten von etwaigen Änderungen in den Zellen.

23. Das Verfahren zur Prüfung der zytologische Wirkung einer Testsubstanz gemäß Anspruch 22, dadurch gekennzeichnet, daß es sich bei den Zellen um blutbildende Zellen handelt.

24. Das Verfahren zur Prüfung der Zytotoxität einer Testsubstanz gemäß Anspruch 23, dadurch gekennzeichnet, daß es sich bei den Zellen um Melanozyten und Keratinozyten handelt.

25. Das Verfahren zur Prüfung der Zytotoxität einer Testsubstanz gemäß Anspruch 23, dadurch gekennzeichnet, daß es sich bei den Zellen um Leberzellen handelt.

26. Ein Verfahren zum Nachweis von metastatischen Zellen, umfassend:
(a) Gewinnen einer Knochenmarkzell-, Hautzell- oder Leberzellprobe;
(b) Beimpfen eines lebenden Stromagewebes gemäß einem der Ansprüche 1 bis 6 mit Zellen aus der Probe;
(c) Kultivieren des beimpften lebenden Stromagewebes in einem Nährmedium, so daß die beimpften Zellen wachsen;
und
(d) Ermitteln des Vorhandenseins von metastatischen Zellen in der gezüchteten Zellkultur.

27. Das Verfahren zum Nachweis von metastatischen Zellen gemaß Anspruch 27, dadurch gekennzeichnet, daß es sich bei den Zellen um blutbildende Zellen handelt.

28. Das Verfahren zum Nachweis von metastatischen Zellen gemäß Anspruch 27, dadurch gekennzeichnet, daß es sich bei den Zellen um Melanozyten und Keratinozyten handelt.

29. Das Verfahren zur Diagnose einer Chromosomenanomalie gemäß Anspruch 27, dadurch gekennzeichnet, daß es sich bei den Zellen um Leberzellen handelt.

30. Ein Verfahren zur In-vitro-Herstellung eines Stromazellen und von den Stromazellen ausgeschiedene Bindegewebsproteine umfassenden, dreidimensionalen lebenden Stromagewebes, beinhaltend das Züchten des Stromagewebes auf einem biokompatiblen nichfiebenden dreidimensionalen Träger, wobei die Stromazellen auf dem Träger so festhaften und den Träger so umschließen, daß eine dreidimensionale Struktur entsteht.

31. Das Verfahren gemäß Anspruch 30, dadurch gekennzeichnet, daß es sich bei den Stromazellen um Fibroblasten handelt.

32. Das Verfahren gemäß Anspruch 30, dadurch gekennzeichnet, daß es sich bei den Stromazellen um eine Kombination aus Fibroblasten und Endothelzellen, Makrophagen, Retikulumzellen, mononukleären Blutzellen oder Fettzellen handelt.

33. Das Verfahren gemäß einem der Ansprüche 30 bis 32, dadurch gekennzeichnet, daß der Träger mit Kollagen vorbeschichtet ist.

34. Das Verfahren gemäß einem der Ansprüche 30 bis 33, dadurch gekennzeichnet, daß es sich bei dem Träger um ein Netz handelt.

35. Das Verfahren gemäß Anspruch 34, dadurch gekennzeichnet, daß das Netz aus Nylon besteht.

## Revendications

1. Tissu tridimensionnel vivant de stroma ("tissu stromal") préparé "in vitro", comprenant des cellules de stroma et des protéines de tissu conjonctif sécrétées par les cellules du stroma, cultivées sur un support tridimensionnel biocompatible et non vivant, tissu dans le cas duquel les cellules de stroma sont fixées sur le support qu'elles entourent pour former une structure tridimensionnelle.

2. Tissu de stroma vivant selon la revendication 1, dans lequel les cellules de stroma sont des fibroblastes.

3. Tissu de stroma vivant selon la revendication 1, dans lequel les cellules du stroma constituent une combinaison de fibroblastes et de cellules endothéliales, de macrophages, de cellules réticulaires, de globules sanguins mononucléaires ou d'adipocytes.

4. Tissu de stroma vivant selon l'une quelconque des revendications précédentes, dans lequel le support est prérevêtu de collagène.

5. Tissu de stroma vivant selon l'une quelconque des revendications précédentes, dans lequel le support est une toile.

6. Tissu de stroma vivant selon la revendication 5, dans lequel la toile est formée de "Nylon".

7. Culture tridimensionnelle de cellules comprenant des cellules cultivées sur un tissu de stroma vivant selon l'une quelconque des revendications précédentes, les cellules étant des cellules de la moelle d'os, de la peau ou du foie.

8. Culture de moelle osseuse tridimensionnelle, comprenant des cellules hématopoïétiques cultivées sur un tissu de stroma vivant selon l'une quelconque des revendications 1 à 6.

9. Culture de peau tridimensionnelle, comprenant des mélanocytes et des kératinocytes cultivés sur un tissu de stroma vivant selon l'une quelconque des revendications 1 à 6.

10. Culture de peau tridimensionnelle selon la revendication 9, dans laquelle les cellules de stroma sont confluentes.

11. Culture de foie tridimensionnelle, comprenant des hépatocytes cultivés sur un tissu de stroma vivant selon l'une quelconque des revendications 1 à 6.

12. Procédé pour cultiver des cellules en verrerie de laboratoire ("in vitro"), ce procédé comprenant :
(a) l'inoculation de cellules de la moelle osseuse, de la peau ou du foie à un tissu de stroma vivant selon l'une quelconque des revendications 1 à 6 ; et
(b) la culture du tissu de stroma vivant ainsi inoculé pour la croissance des cellules inoculées.

13. Procédé pour cultiver "in vitro" des cellules de moelle d'os, comprenant :
(a) l'inoculation de cellules hématopoïétiques à un tissu de stroma vivant selon l'une quelconque des revendications 1 à 6 ; et
(b) la culture du tissu de stroma vivant ayant reçu cette inoculation, de façon que croissent les cellules inoculées.

14. Procédé pour cultiver "in vitro" des cellules de la peau, ce procédé comprenant :
(a) l'inoculation de mélanocytes et de kératinocytes à du tissu de stroma vivant selon l'une quelconque des revendications 1 à 6 ; et
(b) la culture du tissu de stroma vivant ayant reçu cette inoculation, pour que les cellules inoculées croissent.

15. Procédé selon la revendication 14, dans lequel le tissu de stroma vivant comprend des cellules confluentes de stroma.

16. Procédé pour cultiver "in vitro" des cellules du foie, ce procédé comprenant :
(a) l'inoculation d'hépatocytes à du tissu de stroma vivant, selon l'une quelconque des revendications 1 à 6 ; et
(b) la culture du tissu de stroma vivant ayant reçu cette inoculation, de façon que les cellules inoculées croissent.

17. Utilisation de cellules de moelle d'os, de peau ou de foie, cultivées "in vitro", pour la fabrication d'un transplant ; dans laquelle la culture comprend :
(a) l'inoculation de cellules de moelle d'os, de peau ou de foie à du tissu de stroma vivant selon l'une quelconque des revendications 1 à 6 ; et
(b) la culture du tissu de stroma vivant ayant reçu cette inoculation, pour que les cellules inoculées croissent.

18. Utilisation selon la revendication 17, dans laquelle les cellules sont destinées à de la transplantation de cellules de moelle d'os, préparées par culture de cellules hématopoïétiques "in vitro", de sorte que les cellules hématopoïétiques croissent.

19. Utilisation selon la revendication 17 de cellules de la peau, préparées par culture de mélanocytes et de kératinocytes "in vitro", de sorte que les mélanocytes et les kératinocytes croissent.

20. Utilisation selon la revendication 17 de cellules de foie, préparées par la culture d'hépatocytes "in vitro", pour la fabrication d'un transplant.

21. Utilisation de tissu de stroma vivant selon l'une quelconque des revendications 1 à 6 pour la fabrication d'un transplant.

22. Procédé pour vérifier la cytotoxicité d'une substance d'essai, le procédé comprenant :
(a) l'exposition d'une culture tridimensionnelle de cellules à la substance d'essai, la culture tridimensionnelle de cellules comprenant des cellules de moelle d'os, de peau ou de foie dont la croissance a lieu sur un tissu de stroma vivant selon l'une quelconque des revendications 1 à 6 ; et
(b) la détermination de l'effet de la substance d'essai, par observation de toutes les modifications éventuelles apparaissant dans les cellules.

23. Procédé pour vérifier l'effet cytologique d'une substance d'essai selon la revendication 22, dans lequel les cellules sont des cellules hématopoïétiques.

24. Procédé pour vérifier la cytotoxicité d'une substance d'essai, selon la revendication 23, dans lequel les cellules sont des mélanocytes et des kératinocytes.

25. Procédé pour vérifier la cytotoxicité d'une substance d'essai, selon la revendication 23, dans lequel les cellules sont des hépatocytes.

26. Procédé pour déceler des cellules de métastases, comprenant :
(a) l'obtention d'un échantillon de cellules de moelle d'os, de peau ou de foie ;
(b) l'inoculation de cellules provenant de l'échantillon à un tissu de stroma vivant selon l'une quelconque des revendications 1 à 6 ;
(c) la culture du tissu de stroma vivant, ayant reçu cette inoculation, dans un milieu nutritif de façon que les cellules inoculées croissent ; et
(d) la détermination de la présence de cellules de métastases dans les cellules dont la croissance a eu lieu dans cette culture.

27. Procédé pour déceler des cellules de métastases selon la revendication 26, dans lequel les cellules sont des cellules hématopoïétiques.

28. Procédé pour déceler des cellules de métastases selon la revendication 27, dans lequel les cellules sont des ménalocytes et des kératinocytes.

29. Procédé pour poser le diagnostic d'une anomalie chromosomique selon la revendication 27, dans lequel les cellules sont des hépatocytes.

30. Procédé pour préparer "in vitro" un tissu de stroma vivant tridimensionnel, comprenant des cellules de stroma et des protéines de tissu conjonctif sécrétées par les cellules du stroma, ce procédé comprenant la culture du tissu de stroma sur un support tridimensionnel, biocompatible et non vivant, ce qui provoque la fixation des cellules du stroma sur le support qu'elles encerclent pour former une structure tridimensionnelle.

31. Procédé selon la revendication 30, dans lequel les cellules de stroma sont les fibroblastes.

32. Procédé selon la revendication 30, dans lequel les cellules de stroma constituent une combinaison de fibroblastes et de cellules endothéliales, de macrophages, de cellules réticulaires, de globules mononucléaires du sang ou d'adipocytes.

33. Procédé selon l'une quelconque des revendications 30 à 32, dans lequel le support est prérevêtu par du collagène.

34. Procédé selon l'une quelconque des revendications 30 à 33, dans lequel le support est une toile.

35. Procédé selon la revendication 34, dans lequel la toile est formée de "Nylon".
